(19) Europäisches Patentamt
European Patent Office
Office européen des brevets

(11) **EP 3 491 999 B1**

(12) **FASCICULE DE BREVET EUROPEEN**

(45) Date de publication et mention
de la délivrance du brevet:
**20.09.2023 Bulletin 2023/38**

(21) Numéro de dépôt: **18184560.3**

(22) Date de dépôt: **19.07.2018**

(51) Classification Internationale des Brevets (IPC):
**A61B 5/00** *(2006.01)*  **A61B 5/024** *(2006.01)*
**A61B 5/1455** *(2006.01)*

(52) Classification Coopérative des Brevets (CPC):
**A61B 5/14551; A61B 5/02416; A61B 5/7221;
A61B 5/725;** A61B 5/721

(54) **DISPOSITIF D'ÉSTIMATION DE LA SPO2 ET MÉTHODE D'ÉSTIMATION DE LA SPO2**

VORRICHTUNG ZUM SCHÄTZEN DES SPO2-WERTS, UND SCHÄTZMETHODE DES SPO2-WERTS

DEVICE FOR ESTIMATING SPO2 AND METHOD OF ESTIMATING SPO2

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.07.2017 FR 1756904**

(43) Date de publication de la demande:
**05.06.2019 Bulletin 2019/23**

(73) Titulaire: **Commissariat à l'Energie Atomique et
aux Energies
Alternatives
75015 Paris (FR)**

(72) Inventeur: **JALLON, Pierre
38700 CORENC (FR)**

(74) Mandataire: **Brevalex
Tour Trinity
1 B Place de la Défense
92400 Courbevoie (FR)**

(56) Documents cités:
**EP-A1- 1 611 847      US-A1- 2013 079 601
US-A1- 2017 156 593      US-B1- 6 725 074**

## Description

### DOMAINE TECHNIQUE ET ÉTAT DE LA TECHNIQUE ANTÉRIEURE

La présente invention se rapporte à un dispositif d'estimation de l'indicateur SpO2 et à un procédé d'estimation de l'indicateur SpO2

La SpO2 est un indicateur sur la saturation en oxygène de l'hémoglobine au niveau des capillaires sanguins.

Cet indicateur, la SpO2 ou saturation pulsée en oxygène, permet en particulier de détecter les cas d'hypoxie, c'est-à-dire un manque d'oxygénation des cellules. Cela peut par exemple se produire :

- chez les personnes souffrant de maladies respiratoires chroniques, telles que la bronchopneumopathie chronique obstructive (BPCO),
- chez les personnes souffrant d'apnée du sommeil.

En outre, le suivi de cet indicateur peut être utilisé dans la gestion des urgences médicales et peut être utilisé dans certains sports, par exemple avec le mal des montagnes

De manière très fréquente, cette mesure se fait en disposant un oxymètre de pouls à l'extrémité d'un doigt du patient.

Un oxymètre comporte un dispositif optique mettant en oeuvre deux diodes, l'une émettant dans le rouge et l'autre émettant dans l'infrarouge. Ces diodes éclairent le doigt et le signal optique en retour est utilisé pour calculer la SpO2.

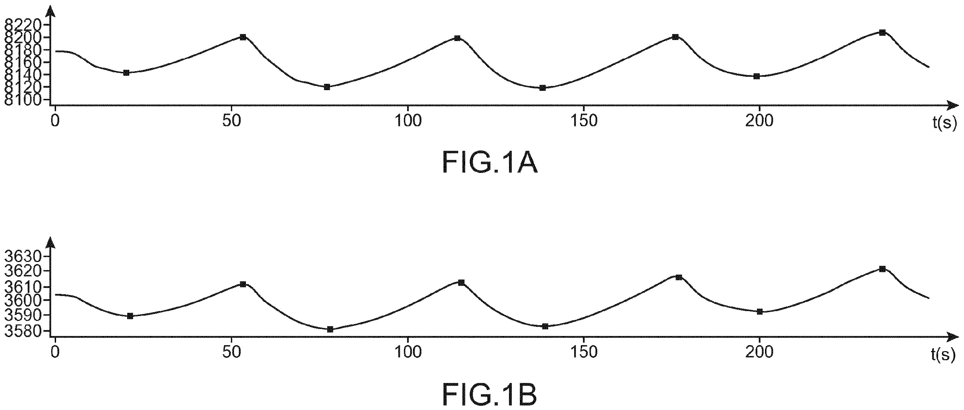

Un exemple de signaux optiques en retour obtenus, appelé signaux PPG (photopléthysmographie) sont représentés sur les figures 1A et 1B. La figure 1A correspond au signal PPG pour la diode émettant dans le rouge, à une longueur d'onde proche de 640 nm, et la figure 1B correspond au signal PPG pour la diode émettant dans l'infrarouge, à une longueur d'onde proche de 940 nm. En ordonnée il s'agit d'une tension proportionnelle à la quantité de lumière captée par le dispositif optique et en abscisse du temps en seconde.

Le critère RR est alors calculé, désigné ratio des ratios, qui est défini comme suit :

$$RR = \frac{\frac{AC_x}{DC_x}}{\frac{AC_y}{DC_y}}$$

Avec :

- $AC_x$ l'amplitude du signal PPG à la longueur d'onde $x$ nm,
- $DC_x$ la valeur minimale (ou moyenne) du signal PPG à la longueur d'onde x nm.
- $AC_y$ l'amplitude du signal PPG à la longueur d'onde y nm,
- $DC_y$ la valeur minimale (ou moyenne) du signal PPG à la longueur d'onde y nm.

La Sp02 est ensuite calculée comme suit :

$$Sp02 = A - B \cdot RR$$

Avec A et B des constantes dépendantes de la position du capteur sur le corps. Ces constantes sont communes à l'ensemble de la population.

Cette méthode d'estimation de la SpO2 suppose que les signaux PPG aient sensiblement l'allure des signaux PPG des figures 1A et 1B. Ceci est souvent le cas pour un système porté au doigt, mais pour un système de type patch ou bracelet, le rapport signal sur bruit peut être moins favorable et rendre l'estimation incorrecte.

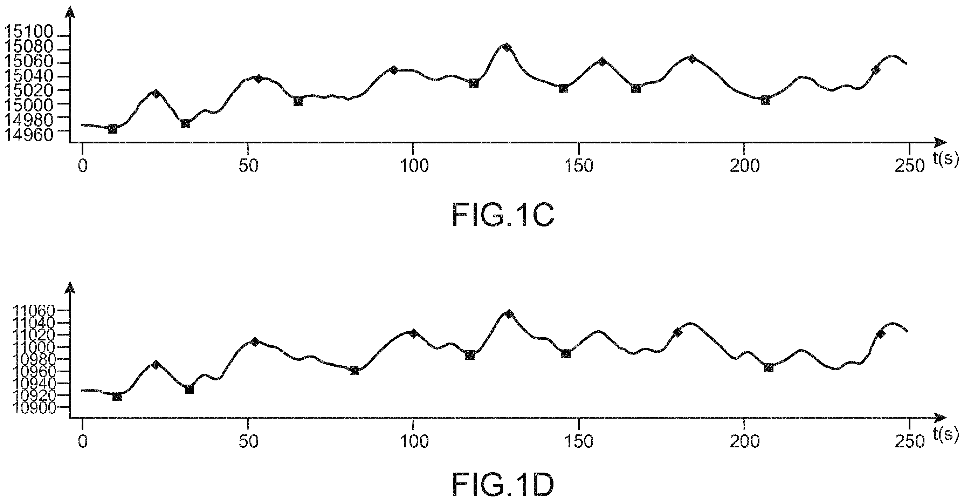

Sur les figures 1C et 1D, on peut des exemples de signaux en retour acquis avec un dispositif porté au poignet. On constate que les signaux sont plus bruités.

Par ailleurs, le mouvement du patient, et en particulier du membre portant l'oxymètre peut perturber de manière importante les signaux PPG.

Or, on cherche à pouvoir placer un oxymètre ailleurs qu'à l'extrémité d'un doigt, par exemple on peut souhaiter le disposer au niveau du poignet, du bras ou du front.

Des méthodes ont été mises au point pour compenser les artefacts dus au mouvement dans les signaux PPG, par exemple le document S. Liu & al, « Using a Kalman filter to compensate for PPG artefacts when monitoring CT examination », IEEE 2009 décrit une telle méthode de compensation des artéfacts de mouvement dans le cadre d'examens de tomodensitométrie. Cependant cette méthode a pour effet de reconstruire l'onde des signaux optiques, ce qui modifie le signal. Or l'estimation de la SpO2 est basée sur la forme d'onde. Toute modification du signal peut alors fausser l'estimation de la SpO2.

Les documents US2013/079601, EP 1 611 847, US 6 725 074 et US2017/156593 décrivent différents dispositifs d'évaluation de la SpO2 d'un être vivant.

### EXPOSÉ DE L'INVENTION

C'est par conséquent un but de la présente invention d'offrir un dispositif d'estimation de la SpO2 et une méthode d'estimation de la SpO2 offrant une bonne estimation de la SpO2 tout en offrant la possibilité de disposer l'oxymètre ailleurs qu'à l'extrémité d'un doigt du patient.

Le but de la présente invention est atteint par un dispositif d'estimation de la SpO2 comportant un oxymètre pour acquérir des signaux PPG dans le rouge et l'infrarouge et une unité de traitement pour traiter les signaux PPG, l'unité de traitement étant telle qu'elle détermine un indicateur de qualité de la valeur du ratio des ratio calculée à partir des mesures, cet indicateur permettant de qualifier le niveau de fiabilité du ratio des ratios calculé et déterminer dans quelle mesure celui-ci peut être utilisé pour estimer le ratio des ratios.

Si son niveau de fiabilité est élevé, la valeur calculée du ratio des ratios influe de manière importante sur la valeur estimée du ratio des ratios et donc sur la valeur de la SpO2. Plus le niveau de fiabilité est faible, moins la valeur calculée du ratio des ratios a d'influence, voire elle n'est pas prise en compte.

Contrairement aux méthodes de l'état de la technique qui réduisent, voire suppriment le bruit du signal PPG et modifient donc la forme d'onde, la méthode selon l'invention construit un indicateur de qualité qui permet de déterminer la fiabilité des mesures, et de décider comment elles participent à l'estimation du ratio des ratios et donc au calcul de la Sp02, sans modifier la forme d'onde des signaux PPG.

Par exemple, l'indicateur de qualité est un indicateur sur la forme d'onde du signal PPG qui reflète l'adéquation du signal PPG pour estimer le ratio des ratios. Cet indicateur est ensuite intégré dans un système de moyenne adaptatif du ratio des ratios.

Selon l'invention, cet indicateur de qualité est déterminé à partir d'un indicateur de validité sur le temps et d'un indicateur de stabilité du signal.

De manière très avantageuse, on peut utiliser au moins un indicateur d'état du patient, par exemple calculé sur la base des signaux émis par un ou plusieurs accéléromètres. Lorsque la valeur de l'indicateur est considérée comme correspondant à un patient en mouvement, il peut être décidé de ne pas acquérir de signaux PPG qui seraient peu ou pas exploitables, les diodes sont alors éteintes, ce qui permet d'optimiser la consommation.

Dans un exemple avantageux, un filtre de Kalman est mis en oeuvre pour estimer le ratio des ratios.

La présente invention a alors pour objet un dispositif d'évaluation de la SpO2 d'un être vivant selon la revendication 1.

Selon l'invention, les moyens de détermination d'un indicateur de qualité des signaux PPG sont configurés pour déterminer un indicateur de validité temporel de chacun des signaux PPG, un indicateur de stabilité de chacun des signaux PPG et pour déterminer à partir des indicateurs de validité et des indicateurs de stabilité, l'indicateur de qualité.

Par exemple, l'indicateur de qualité est choisi égal à la valeur maximale parmi les valeurs des indicateurs de validité et les indicateurs de stabilité.

Selon un exemple de réalisation, les moyens pour vérifier la périodicité de chacun des signaux PPG sont configurés pour calculer la densité spectrale de chacun des signaux PPG, vérifier l'existence d'un maximum local unique et la valeur de l'amplitude dudit maximum local par rapport à un seuil donné.

Dans un exemple avantageux, le dispositif d'estimation de la SpO2 comporte des moyens pour générer un signal relatif à un mouvement dudit être vivant et des moyens pour suspendre l'activité de l'oxymètre lorsque le signal relatif à un mouvement est considéré comme indiquant que l'être vivant est en mouvement. Les moyens pour générer un signal relatif à un mouvement sont par exemple un accéléromètre.

La présente invention a également pour objet une méthode d'estimation de la SpO2 d'un être vivant à partir d'au moins deux signaux photopléthysmographiques, dit signaux PPG, l'un dans le rouge et l'autre dans l'infrarouge, comportant les étapes :

    a) Vérification de la périodicité de chacun des signaux.
    b) Calcul d'un indicateur de qualité d'un coefficient appelé ratio des ratios.
    c) Estimation du ratio des ratios et calcul de la SpO2.

L'étape b) comporte également le calcul d'un indicateur de validité temporel de chacun des signaux PPG, d'un indicateur de stabilité de chacun des signaux PPG et de détermination à partir des indicateurs de validité et des indicateurs de stabilité, l'indicateur de qualité($I_R$). L'indicateur de validité $V_{T,x}$ est égal à :

$$V_{T,x} = \max_i abs\left(\frac{T_{i,x} - T_s}{T_s}\right)$$

avec $T_i$, $x$ le temps entre deux minimums locaux successifs du signal PPG à la longueur d'onde $x$, et $T_s$ la période moyenne dudit signal

L'indicateur de stabilité est égal à

$$V_{A,x} = \max_i abs\left(\frac{d_{i,x} - d_{i-1,x}}{d_{i-1,x}}\right)$$

avec $d_{i,x}$ l'amplitude du minimum local pour la période #i correspondant à la longueur d'onde x.

Par exemple, l'indicateur de qualité est choisi égal à la valeur maximale parmi les valeurs des indicateurs de validité et les indicateurs de stabilité.

L'étape c) peut mettre en oeuvre un modèle d'estimation du ratio des ratios utilisant des paramètres dont les valeurs sont fixées en fonction de la valeur de l'indicateur de qualité.

Avantageusement l'étape c) met en oeuvre un filtre de Kalman. Le filtre de Kalman suit par exemple le modèle :

$$\widehat{RR}_k = \widehat{RR}_{k-1} + n_x$$

$$RR_k = a_k\widehat{RR}_k + n_y$$

*Avec :*

- $RR_k$ le ratio des ratios calculé à parti des valeurs AC et DC mesurées sur le signal PPG,
- $\widehat{RR}_k$ le ratio des ratios estimé et qui sera utilisé pour le calcul de la Sp02,
- $n_x$ un bruit blanc gaussien centré de variance $\sigma_x = 0.005$,
- $n_y$ un bruit blanc gaussien centré de variance $\sigma_y(k)$,

et dans laquelle les valeurs de $a_k$ et de $\sigma_y(k)$ sont choisis en fonction de la valeur de l'indicateur de qualité.

Par exemple, on applique :

- Si l'indicateur de qualité <0,1, $\sigma_y(k) = 0,05$ et $a_k = 1$,
- Si 0,1 < l'indicateur de qualité <0,2, $\sigma_y(k) = 0.2$ et $a_k = 1$
- Si 0,2<l'indicateur de qualité <0,4, $\sigma_y(k) = 0,5$ et $a_k = 1$
- Si 0,4 < l'indicateur de qualité <0,8, $\sigma_y(k) = 1,0$ et $a_k = 0$
- Si l'indicateur de qualité > 0,8, $\sigma_y(k) = 2,0$ et $a_k = 0$

L'étape a) peut comporter le calcul de la densité spectrale de puissance de chacun des signaux, la détection d'un seul maximum local et la vérification que ledit maximum local a une amplitude au moins égal à un seuil donné.

Lors de l'étape a), il peut être prévu de déterminer le rythme cardiaque.

La méthode d'estimation de la SpO2 peut comporter l'enregistrement des signaux PPG préalablement à l'étape a).

**BRÈVE DESCRIPTION DES DESSINS**

La présente invention sera mieux comprise sur la base de la description qui va suivre et des dessins en annexe sur lesquels:

- les figures 1A et 1B sont des représentations graphiques d'un exemple de signaux PPG dans le rouge et dans l'infrarouge respectivement dans le cas d'une mesure à l'extrémité d'un doigt en fonction du temps en seconde,
- les figures 1C et 1D sont des représentations graphiques d'un exemple de signaux PPG dans le rouge et dans l'infrarouge respectivement dans le cas d'une mesure à un poignet en fonction du temps,
- la figure 2 est une représentation schématique d'un dispositif d'estimation de la SpO2 selon un exemple de réalisation de la présente invention,
- la figure 3A est une représentation graphique de signaux PPG dans le rouge et dans l'infrarouge dans le cas d'un oxymètre sur le poignet d'un patient,
- la figure 3B est une représentation graphique du logarithme de la DSP calculée du signal PPG correspondant à la longueur d'onde rouge de la figure 3A, en fonction de l'index des coefficients de Fourier (proportionnel à la fréquence en Hz),

- la figure 4A est une représentation graphique d'autres signaux PPG dans le rouge et dans l'infrarouge dans le cas d'un oxymètre sur le poignet d'un patient,
- la figure 4B est une représentation graphique du logarithme de la DSP calculée du signal PPG correspondant à la longueur d'onde rouge de la figure 3A de la figure 4A en fonction de l'index des coefficients de Fourier (proportionnel à la fréquence en Hz),
- la figure 5A est une représentation graphique de la SpO2 calculée grâce au dispositif selon l'invention, dans le cas d'un oxymètre disposé sur front du patient,
- la figure 5B est une représentation graphique de la SpO2 calculée grâce à un dispositif de l'état de la technique, dans le cas d'un oxymètre disposé sur l'extrémité d'un doigt du patient,
- la figure 6A est une représentation graphique de la SpO2 calculée grâce au dispositif selon l'invention, dans le cas d'un oxymètre disposé sur un avant-bras du patient,
- la figure 6B est une représentation graphique de la SpO2 calculée grâce à un dispositif de l'état de la technique, dans le cas d'un oxymètre disposé sur l'extrémité d'un doigt du patient,
- la figure 7A est une représentation graphique de la SpO2 calculée grâce au dispositif selon l'invention, dans le cas d'un oxymètre disposé sur un poignet du patient,
- la figure 7B est une représentation graphique de la SpO2 calculée grâce à un dispositif de l'état de la technique, dans le cas d'un oxymètre disposé sur l'extrémité d'un doigt du patient,
- la figure 8A est une représentation graphique de la SpO2 calculée d'autres signaux PPG grâce au dispositif selon l'invention, dans le cas d'un oxymètre disposé sur un poignet du patient,
- la figure 8B est une représentation graphique de la SpO2 calculée grâce à un dispositif de l'état de la technique, dans le cas d'un oxymètre disposé sur l'extrémité d'un doigt du patient,
- les figures 9A et 9B sont des représentations graphiques de mesures PPG dans le rouge et l'infrarouge respectivement obtenues au niveau d'un poignet, et le niveau de SpO2 déterminé grâce à la méthode selon l'invention.

**EXPOSÉ DÉTAILLÉ DE MODES DE RÉALISATION PARTICULIERS**

Dans la présente demande, on entend par « ratio des ratios calculé », le ratio des ratios calculé à partir des mesures directes sur les signaux PPG. On entend par « ratio des ratios » estimé, le ratio des ratios qui est utilisé pour calculer la SpO2.

L'indicateur de saturation pulsée en oxygène sera désigné SpO2 dans la suite de la demande.

Le dispositif et la méthode d'estimation de la SpO2 selon l'invention peuvent être utilisés pour tout être humain et tous les animaux pour lesquels on peut utiliser un oxymètre, les êtres humains et ces animaux seront désignés les êtres vivants.

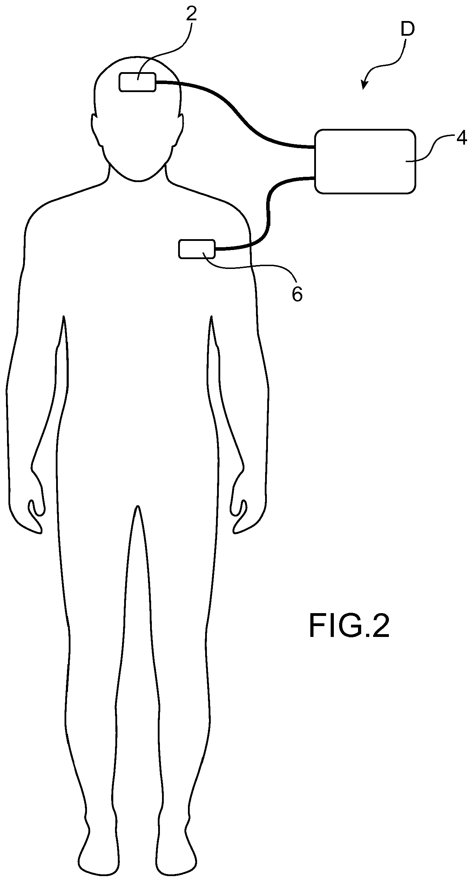

Sur la figure 2, on peut voir une représentation schématique d'un dispositif d'estimation de la SpO2 selon un exemple de réalisation de l'invention.

Le dispositif D comporte un oxymètre 2 et une unité de traitement 4 reliée à l'oxymètre. L'unité de traitement 4 comporte des moyens pour traiter les signaux fournis par l'oxymètre et générer une estimation de la Spo2.

L'oxymètre fournit des signaux PPG.

Un oxymètre est bien connu de l'homme du métier et ne sera pas décrit en détail. Il comporte un dispositif optique à deux diodes, l'une émettant dans le rouge, par exemple à la fréquence de 660 nm, et l'autre dans l'infrarouge par exemple à une fréquence de 940 nm. Le dispositif optique fonctionne en réflexion.

Dans l'exemple représenté, l'oxymètre est disposé sur le front du patient. L'oxymètre pourrait être disposé sur une autre partie du corps du patient, par exemple un avant-bras, un poignet, à l'extrémité d'un doigt. La forme de l'oxymètre est alors adaptée en fonction de la partie du corps sur laquelle il va être disposé.

Par exemple, le dispositif D est portatif ou nomade et est alimenté par une batterie.

Le calcul de la SpO2 est effectuée à partir du calcul du ratio des ratios qui est déterminé à partir des signaux PPG à la longueur d'onde correspondant au rouge et à une longueur d'onde correspondant à de l'infrarouge.

La Sp02 est calculée comme suit :

$$Sp02 = A - B \cdot RR$$

Avec A et B des constantes dépendantes de la position du capteur sur le corps mais communes à l'ensemble de la population.

RR le ratio des ratios est défini comme suit :

$$RR = \frac{\frac{AC_x}{DC_x}}{\frac{AC_y}{DC_y}}$$

Avec :

- $AC_x$ est l'amplitude du signal PPG à la longueur d'onde $\times$ nm (dans le rouge)
- $DC_x$ est la valeur minimale (ou moyenne) du signal PPG à la longueur d'onde $\times$ nm (dans le rouge).
- $AC_y$ est l'amplitude du signal PPG à la longueur d'onde y nm (dans l'infrarouge)
- $DC_y$ est la valeur minimale (ou moyenne) du signal PPG à la longueur d'onde y nm (dans l'infrarouge).

Les signaux PPG sont enregistrés par exemple à l'aide d'un oxymètre monté sur l'extrémité d'un doigt, sur un poignet par exemple au moyen d'un bracelet, ou sur le front d'un patient. Ces mesures peuvent être faites lors d'une phase de sommeil du patient ou lors d'une phase éveillée.

Les moyens de traitement et d'estimation de la SpO2 sont tels qu'ils mettent en oeuvre la méthode d'estimation selon l'invention, les signaux dans le rouge et dans l'infrarouge fournis par l'oxymètre 2.

La méthode d'estimation comporte au moins les étapes suivantes :

a) Vérification de la périodicité des deux signaux.
b) Calcul d'un indicateur de qualité du ratio des ratios.
c) Estimation du ratio des ratios et calcul de la SpO2.

Cette méthode est mise en oeuvre par un ordinateur pouvant traiter les mesures PPG et leur appliquer la méthode selon l'invention.

Lors de l'étape a), les signaux PPG sont traités pour vérifier si ceux-ci sont périodiques. Pour cela on détermine par exemple la fréquence cardiaque et on vérifie sa périodicité. La courbe PPGR1 correspond au signal PPG dans le rouge et la courbe PPGIR1 correspond au signal PPG dans l'infrarouge.

Par exemple, lors de cette étape a), on découpe chacun des signaux en fenêtres temporelles. Ces fenêtres temporelles sont choisies de manière à représenter quelques périodes de battements cardiaques mais de manière stable c'est-à-dire peu sensible à des phénomènes de variabilité de rythme cardiaque. Par exemple, une taille de fenêtre temporelle correspondant à 5 secondes convient.

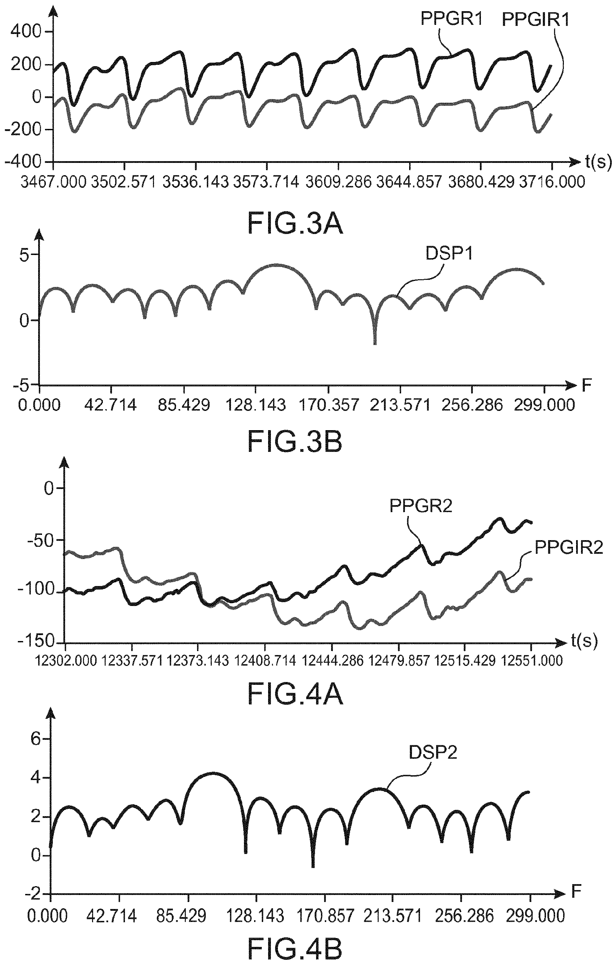

On peut centrer les signaux PPG sur la fenêtre temporelle (figure 3A).

On peut de plus normaliser les signaux PPG sur la fenêtre temporelle.

On réalise ensuite une analyse spectrale des signaux afin de décider s'ils peuvent être considérés comme périodiques et donc être utilisés éventuellement pour calculer le SpO2.

L'analyse spectrale peut consister à calculer la densité spectrale de puissance ou DSP, le logarithme de la DSP du signal PPG correspondant à la longueur d'onde rouge est représenté sur la figure 3B.

Le logarithme de la DSP, DSP1, est ensuite traité pour identifier les maximums locaux de cette DSP et supprimer les éventuels maximums locaux parasites. Ces maximums locaux parasites correspondent par exemple aux lobes des maxima principaux.

Après ce traitement on vérifie ensuite s'il ne reste qu'un seul maximum local et si son amplitude est supérieure à un certain seuil. Si c'est le cas on en déduit que le signal PPG est effectivement périodique. Le critère des propriétés fréquentielles du signal est alors validé. Par exemple sur la figure 3B, le seuil est fixé à 4, et l'amplitude du maximum local est 4,1. Le critère des propriétés fréquentielles est donc validé.

Ce traitement et la vérification de la périodicité du signal sont réalisés pour les signaux PPGR1 et PPGIR1.

A la fin de cette étape, on connaît la fréquence cardiaque du patient et on peut en déduire la période moyenne des signaux PPGR et PPGIR.

Sur les figures 4A et 4B, on peut voir un autre exemple de traitement des signaux PPG. Sur la figure 4A on peut voir un exemple de signaux PPG, désigné PPGR2 et PPGIR2 et sur la figure 4B on peut voir représenté le logarithme de la DSP, DSP2, du signal PPG rouge. L'amplitude du seul maximum local est égale à 4,1, elle est supérieure au seuil de 4. Le critère de périodicité est alors également rempli.

Lors de l'étape b), un indicateur de qualité du ratio des ratios est calculé.

Pour cela, on introduit quelques points significatifs sur signal PPG :

- $T_S = \frac{60}{F_c}$ où $F_c$ est la fréquence cardiaque en minute. $T_S$ est la durée en seconde d'une période du signal PPG (à noter que les signaux rouge et infrarouge partagent cette même période).

- P est le nombre de périodes « complètes » observées dans le signal :

$$P = \left\lfloor T \cdot \frac{F_c}{60} \right\rfloor = \left\lfloor \frac{T}{T_s} \right\rfloor$$

Où T est la taille de la fenêtre d'analyse en secondes (T=5s par exemple).

Par exemple, sur la figure 1A, $P = 4$. Sur la figure 3A, $P = 8$.

- Les extrema locaux des signaux PPG :

  ○ v $i \in [0, P - 1]$, $d_{i,x}$ est l'amplitude du minimum local pour la période #i du signal PPG correspondant à la longueur d'onde $x$.
  ○ b' $i \in [0, P - 1]$, $\alpha_{i,x}$ est l'instant auquel le minimum local pour la période #i du signal PPG correspondant à la longueur d'onde $x$ est atteint.
  ○ b' $i \in [0, P - 1]$, $D_{i,x}$ est l'amplitude du maximum local pour la période #i du signal PPG correspondant à la longueur d'onde $x$.

- On note de plus :

  ○ b' $i \in [0, P - 2]$, $T_{i,x} = \alpha_{i+1,x} - \alpha_{i,x}$ : le temps entre deux minimums locaux successifs du signal PPG correspondant à la longueur d'onde x.

Selon l'invention, on détermine un indicateur de validité sur le temps désigne $V_T$, qui est défini par :

$$V_{T,x} = \max_i abs\left(\frac{T_{i,x} - T_s}{T_s}\right)$$

Cet indicateur donne une information sur la variation de la période locale par rapport à la période moyenne. Plus la valeur de $V_{T,x}$ est faible, meilleure est la forme du signal pour l'estimation du ratio des ratios.

Selon l'invention, on détermine également un indicateur de stabilité $V_A$ du signal défini comme suit :

$$V_{A,x} = \max_i abs\left(\frac{d_{i,x} - d_{i-1,x}}{d_{i-1,x}}\right)$$

Plus la valeur de $V_{A,x}$ est faible, meilleure est la forme du signal pour l'estimation du ratio des ratios.

Les déterminations des indicateurs $V_{T,x}$ et $V_{A,y}$ permettent de déterminer si les minimums et les maximums locaux des signaux sont bien positionnés.

L'indicateur global de qualité de RR, désigné $I_R$, est défini comme suit :

$$I_R = \max_i\{V_{T,x}, V_{T,y}, V_{A,x}, V_{A,y}\}$$

$I_R$ est choisi égal à la valeur la plus élevée parmi les indicateurs de validité et de stabilité des signaux PPGR et PPGIR, c'est-à-dire que $I_R$ est choisi de sorte à considérer l'indicateur le plus mauvais des signaux PPGR et PPGIR. On se place donc dans la situation la plus « pessimiste », la méthode d'estimation offre donc un haut niveau d'exigence en termes de qualité de signal.

Selon d'autres exemples de réalisation, d'autres indicateurs peuvent être envisagés, par exemple en restreignant les indicateurs à une seule des longueurs d'ondes. Dans d'autres exemples, la fonction « max » peut être remplacée par une norme euclidienne ou toute autre norme, par exemple une somme des carrés des indicateurs, une somme pondérée des indicateurs..., i.e. toute combinaison de tout ou partie de ces critères.

La valeur de l'indicateur global de qualité $I_R$ permet ensuite de choisir des paramètres utilisés lors de l'étape d'estimation du ratio des ratios. Plus la valeur de $I_R$ est élevée, plus le niveau de confiance dans le signal PPG mesuré est faible.

Lors de l'étape c), on estime le ratio des ratios.

Nous disposons des signaux PPGR et PPGIR mesurés mais ces signaux sont bruités et ne peuvent servir directement à calculer de manière fiable le ratio des ratios RR pour calculer la SpO2.

Ce ratio est de préférence calculé comme suit, en réalisant la moyenne sur les P périodes:

$$RR = \frac{1}{P} \sum_{p=0}^{P-1} \frac{\frac{D_{p,x} - d_{p,x}}{d_{p,x}}}{\frac{D_{p,y} - d_{p,y}}{d_{p,y}}}$$

Où x représente les index calculés à partir du signal PPG rouge et y les index calculés à partir du signal PPG infrarouge.

Dans un autre exemple de réalisation, le ratio peut également être calculé comme :

$$RR = \frac{\frac{\frac{1}{P}\Sigma_p(D_{i,x} - d_{i,x})}{\frac{1}{P}\Sigma_p(d_{i,x})}}{\frac{\frac{1}{P}\Sigma_p(D_{i,y} - d_{i,y})}{\frac{1}{P}\Sigma_p(d_{i,y})}}$$

On met en oeuvre un système de moyenne adaptatif du ratio des ratios. De manière avantageuse on utilise un filtre de Kalman.

Il est basé sur le modèle suivant :

$$\widehat{RR}_k = \widehat{RR}_{k-1} + n_x$$

$$RR_k = a_k \widehat{RR}_k + n_y$$

*Avec :*

- $RR_k$ le ratio des ratios calculé à parti des valeurs AC et DC mesurées sur le signal PPG,

- $\widehat{RR}_k$ le ratio des ratios estimé et qui sera utilisé pour le calcul de la Sp02,
- $n_x$ un bruit blanc gaussien centré de variance $\sigma_x$ = 0.005,
- $n_y$ un bruit blanc gaussien centré de variance $\sigma_y(k)$.

Cet algorithme filtre les valeurs du ratio des ratios en fonction de la valeur précédente et de la valeur mesurée.

En fonction de la valeur de l'indicateur $I_R$ déterminée à l'étape b), les paramètres du filtre sont choisis.

Dans un exemple de réalisation,

- Si $I_R$ <0,1, $\sigma_y(k)$ = 0,05 et $a_k$ = 1
- Si 0,1 < $I_R$ <0,2, $\sigma_y(k)$ = 0,2 et $a_k$ = 1
- Si 0,2< $I_R$ <0,4, $\sigma_y(k)$ = 0,5 et $a_k$ = 1
- Si 0,4 < $I_R$ <0,8, $\sigma_y(k)$ = 1,0 et $a_k$ = 0
- Sinon $\sigma_y(k)$ = 2,0 et $a_k$ = 0

Les valeurs de $\sigma_y(k)$ sont choisies de manière relative les uns par rapport aux autres. La plus petite valeur choisie est choisie par rapport à celle de $\sigma_x$. Plus la valeur de $\sigma_y(k)$ est élevée, plus les valeurs de RR calculées sont dispersées et sont donc peu fiables, et inversement plus la valeur de $\sigma_y(k)$ est faible, plus la valeur du ratio des ratios calculé à partir des valeurs mesurées est fiable et peut influer sur la valeur du ratio des ratios estimée. Ainsi, dans l'exemple donné ci-dessus, si $I_R$ >0.4, $\sigma_y(k)$ = 1,0 et $a_k$ = 0, la valeur calculée du ratio des ratios directement à partir des mesures sur le signal n'est alors pas prise en compte dans l'estimation du ratio des ratios. Dans ce cas la valeur estimée de RR est égale à la valeur de RR estimée à l'instant précédent, i.e. $RR_k$ = $RR_{k-1}$.

D'autres stratégies de choix pour les règles de ce filtrage adaptatif sont possibles. Par exemple, en prenant en compte la valeur de RR estimée pour accroître la confiance quand elle correspond à une valeur de Sp02 > 0.95 et la réduire si la valeur est en deçà. Cela permet par exemple de faire converger le système rapidement si la valeur de SpO2 est conforme à la valeur moyenne observée dans la population. A contrario, si la valeur n'est pas dans cette plage classique, le temps de convergence de la mesure sera plus long. Ce type d'approche permet donc de moyenner fortement les artefacts de mesure tout en détectant les cas d'hypoxie prolongés (SpO2 basse) et donc pourrait être adoptée pour des alarmes automatisées basées sur cette mesure.

Dans un autre exemple de réalisation, le jeu de paramètres $I_R$, $\sigma_y(k)$ est adapté en fonction de la position du capteur sur le corps. Par exemple, une mesure au niveau du front est en général plus fiable qu'une mesure sur le sternum. Par conséquent dans le cas de mesures sur le front, la confiance dans les mesures est plus élevée et les paramètres sont moins « protecteurs ». Dans le cas d'une mesure sur le sternum, les paramètres sont choisis pour tenir compte de la fiabilité plus faible des mesures.

Dans un autre exemple de réalisation, d'autres techniques de filtrage adaptatif pourrait être utilisées pour estimer le ratio des ratios. Par exemple des méthodes à base de filtrage particulaire, décrites par exemple dans le document *Kalman and Particle filters,* H. R. B. Orlande and al, The Eurotherm Seminar 94, Advanced Spring School "Thermal Measurements and Inverse Techniques", 5th Edition, Station Biologique de ROSCOFF, June 13-18, 2011.

Ensuite lorsque la valeur du ratio des ratios a été estimée, la valeur de la SpO2 est calculée.

Pour cela, on utilise la formule SpO2 = A-B×RR.

Dans le tableau ci-dessous sont regroupés les exemples de valeurs de A et B en fonction de la position de l'oxymètre sur le corps du patient. Les valeurs de A et B ont été estimées sur une base d'acquisitions de données physiologiques réalisée en milieu clinique.

| POSITION | A | B |
|----------|-----------|-----------|
| BRAS | 117,274882 | 24,735287 |

(suite)

| POSITION | A | B |
|---|---|---|
| DOIGT | 106,897375 | 20,513283 |
| FRONT | 119,030901 | 25,525888 |
| POIGNET | 114,788618 | 20,913078 |

A titre d'exemple nous allons déterminer les valeurs de SpO2 dans le cas des signaux PPG des figures 3A et 4A.

Comme indiqué ci-dessus, les signaux PPG des figures 3A et 4A remplissent le critère de périodicité.

On peut alors déterminer la valeur de $I_R$.

- Dans le cas des signaux de la figure 3A, la valeur de $I_R$. déterminée est égale à 0,04. Celle-ci est inférieure à 0,1, la qualité des signaux est donc très bonne. Les valeurs des paramètres du filtre sont alors choisis comme suit $\sigma_y(k)$ = 0,05 et $a_k$ = 1. La valeur calculée du ratio des ratios sur les signaux influe alors de manière importante sur la valeur estimée des ratios des ratios.
- Dans le cas des signaux de la figure 4A, la valeur de $I_R$. déterminée est égale à 0,34. Celle-ci est comprise entre 0,2 et 0, 4, la qualité des signaux est donc moyenne. Les valeurs des paramètres du filtre sont alors choisies comme suit : $\sigma_y(k)$ = 0,5 et $a_k$ = 1. La valeur calculée du ratio des ratios sur les signaux influe de manière moindre sur la valeur estimée des ratios des ratios.

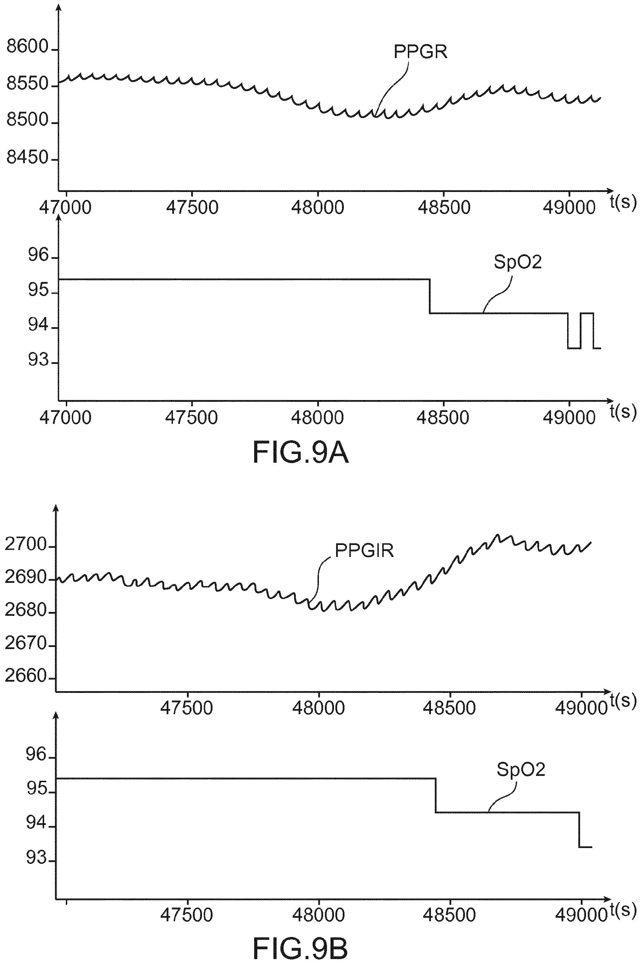

Sur les figures 9A et 9B, on peut voir les courbes des mesures PPG dans le rouge (PPGR) et l'infrarouge (PPGIR) respectivement obtenues au niveau d'un poignet, et le niveau de SpO2 obtenu grâce à la méthode selon l'invention.

Les signaux PPG sont altérés par les mouvements du patient. De manière très avantageuse, on peut prévoir de détecter les mouvements du patient, par exemple à l'aide d'un accéléromètre 6, pour décider de réaliser les mesures des signaux PPG.

Dans l'état de la technique, un accéléromètre est mis en oeuvre et les informations émises par celui-ci sont utilisés pour corriger les signaux PPG afin d'atténuer le bruit dû au mouvement.

Dans un exemple avantageux de l'invention, on décide lorsque les informations fournies par l'accéléromètre, par exemple un accéléromètre trois axes, que les mouvements du patient sont suffisants pour altérer les signaux PPG de sorte qu'ils ne soient pas utilisables, de ne pas les mesurer. L'oxymètre n'est alors pas activé, en particulier les diodes de l'oxymètre ne sont pas allumées, ce qui permet de réduire la consommation électrique de l'oxymètre, ce qui particulièrement intéressant en cas de dispositif portatif ou nomade.

Dans un autre exemple, il peut être décidé d'effectuer les mesures les signaux PPG malgré les mouvements du patient, les signaux de l'accéléromètre peuvent être utilisés pour traiter les signaux PPG par exemple pour en extraire la fréquence cardiaque.

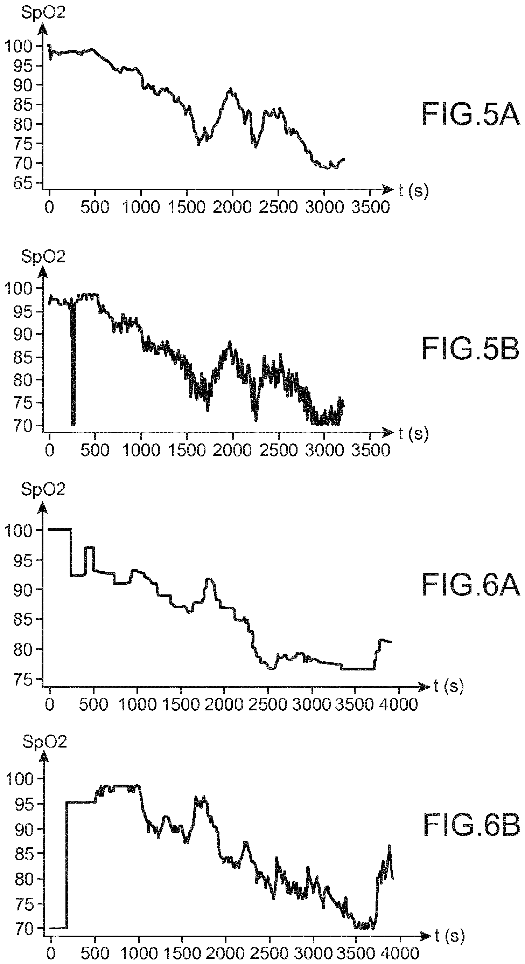

Sur la figure 5A, on peut voir la variation de la SpO2 en fonction du temps en seconde obtenue à partir de la méthode d'estimation selon l'invention, l'oxymètre étant disposé sur le front du patient.

A titre de comparaison, sur la figure 5B on peut voir la variation de la SpO2 obtenue avec un oxymètre monté sur l'extrémité du doigt du patient en utilisant une méthode de l'état de la technique.

Sur la figure 6A, on peut voir la variation de la SpO2 en fonction du temps en seconde obtenue à partir de la méthode d'estimation selon l'invention, l'oxymètre étant disposé sur l'avant-bras du patient. Les plateaux correspondent à des instants lors desquels les signaux mesurées n'ont pas été pris en compte dans l'estimation car l'indicateur $I_R$ était trop élevé, la valeur de RR estimée a été choisie égale à la valeur de RR estimée à l'instant précédent

A titre de comparaison, sur la figure 6B on peut voir la variation de la SpO2 obtenue avec un oxymètre monté sur l'extrémité du doigt du patient en utilisant une méthode de l'état de la technique.

Sur la figure 6A, on peut voir la variation de la SpO2 en fonction du temps en seconde obtenue à partir de la méthode d'estimation selon l'invention, l'oxymètre étant disposé sur l'avant-bras du patient. Les plateaux correspondent aux instants lors desquels les signaux mesurés n'ont pas été pris en compte dans l'estimation car l'indicateur IR était trop élevé, la valeur de RR estimée a été choisie égale à la valeur de RR estimée à l'instant précédent

A titre de comparaison, sur la figure 6B on peut voir la variation de la SpO2 obtenue avec un oxymètre monté sur l'extrémité du doigt du patient en utilisant une méthode de l'état de la technique.

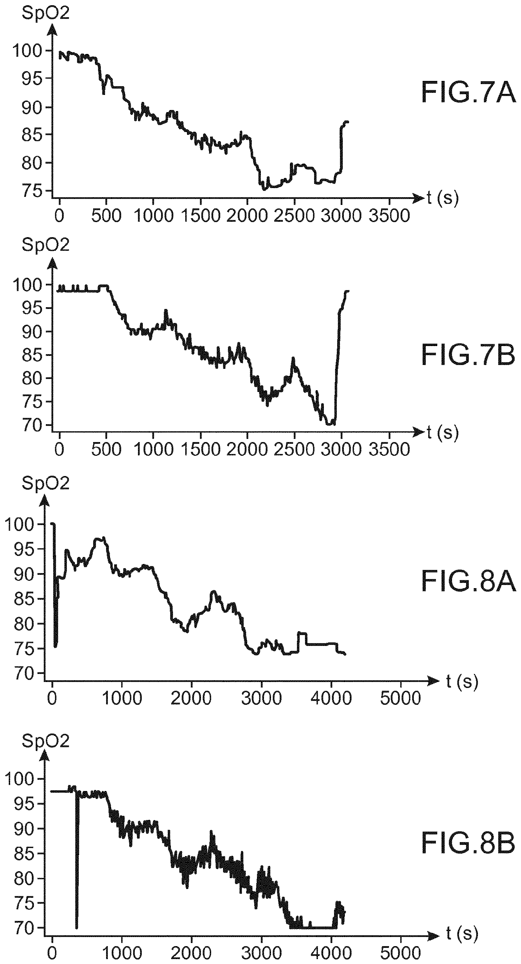

Sur la figure 7A, on peut voir la variation de la SpO2 en fonction du temps en seconde obtenue à partir de la méthode d'estimation selon l'invention, l'oxymètre étant disposé sur le poignet du patient. Les plateaux correspondent à des instants lors desquels les signaux mesurés n'ont pas été pris en compte dans l'estimation car l'indicateur IR était trop élevé, la valeur de RR estimée a été choisie égale à la valeur de RR estimée à l'instant précédent

A titre de comparaison, sur la figure 7B on peut voir la variation de la SpO2 obtenue avec un oxymètre monté sur l'extrémité du doigt du patient en utilisant une méthode de l'état de la technique.

Sur la figure 8A, on peut voir une autre variation de la SpO2 en fonction du temps en seconde obtenue à partir de la méthode d'estimation selon l'invention, l'oxymètre étant disposé sur le poignet du patient. Les plateaux correspondent à des instants lors desquels les signaux mesurés n'ont pas été pris en compte dans l'estimation car l'indicateur $I_R$ était trop élevé, la valeur de RR estimée a été choisie égale à la valeur de RR estimée à l'instant précédent.

A titre de comparaison, sur la figure 8B on peut voir la variation de la SpO2 obtenue avec un oxymètre monté sur l'extrémité du doigt du patient en utilisant une méthode de l'état de la technique.

Grâce à l'invention, on peut obtenir des variations de la SpO2 fiable sur d'autres parties du corps qu'au bout du doigt telles que, par exemple, le front, le poignet ou le sternum.

**Revendications**

1. Dispositif d'évaluation de la SpO2 d'un être vivant, comportant un oxymètre (2) destiné à être fixé sur ledit être vivant, ledit oxymètre étant configuré pour produire au moins deux signaux photopléthysmographiques, dit signaux PPG, l'un dans le rouge et l'autre dans l'infrarouge, et une unité de traitement (4) desdits signaux PPG, ladite unité de traitement (4) comportant des moyens pour vérifier la périodicité de chacun des signaux PPG, des moyens de détermination d'un indicateur de qualité ($I_R$) des signaux PPG, des moyens d'estimation du ratio des ratios (RR), le ratio des ratios (RR) étant défini par :

$$RR = \frac{\frac{AC_x}{DC_x}}{\frac{AC_y}{DC_y}}$$

Avec :

- $AC_x$ l'amplitude du signal PPG à la longueur d'onde × nm,
- $DC_x$ la valeur minimale (ou moyenne) du signal PPG à la longueur d'onde × nm.
- $AC_y$ l'amplitude du signal PPG à la longueur d'onde y nm,
- $DC_y$ la valeur minimale (ou moyenne) du signal PPG à la longueur d'onde y nm,

et des moyens de calcul de la SpO2 à partir de la valeur estimée du ratio des ratios (RR), les moyens d'estimation tenant compte de la valeur de l'indicateur de qualité ($I_R$) dans l'estimation du ratio des ratios (RR),

dans lequel les moyens de détermination d'un indicateur de qualité ($I_R$) des signaux PPG sont configurés pour déterminer un indicateur de validité temporel ($V_{T,x}$) de chacun des signaux PPG, l'indicateur de validité ($V_{T,x}$) étant égal à :

$$V_{T,x} = \max_i abs\left(\frac{T_{i,x} - T_s}{T_s}\right)$$

avec $T_{i,x}$ le temps entre deux minimums locaux successifs du signal PPG à la longueur d'onde x, et $T_s$ la période moyenne dudit signal, un indicateur de stabilité ($V_{A,x}$) de chacun des signaux PPG, l'indicateur de stabilité étant égal à

$$V_{A,x} = \max_i abs\left(\frac{d_{i,x} - d_{i-1,x}}{d_{i-1,x}}\right)$$

avec $d_{i,x}$ l'amplitude du minimum local pour la période #i correspondant à la longueur d'onde x, et pour déterminer à partir des indicateurs de validité et des indicateurs de stabilité, l'indicateur de qualité ($I_R$).

2. Dispositif d'estimation de la SpO2 selon la revendication 1, dans laquelle l'indicateur de qualité ($I_R$) est choisi égal à la valeur maximale parmi les valeurs des indicateurs de validité et les indicateurs de stabilité.

3. Dispositif d'estimation de la SpO2 selon la revendication 1 ou 2, dans lequel les moyens pour vérifier la périodicité de chacun des signaux PPG sont configurés pour calculer la densité spectrale de chacun des signaux PPG, vérifier l'existence d'un maximum local unique et la valeur de l'amplitude dudit maximum local par rapport à un seuil donné.

4. Dispositif d'estimation de la SpO2 selon l'une des revendications 1 à 3, comportant des moyens pour générer un signal relatif à un mouvement dudit être vivant et des moyens pour suspendre l'activité de l'oxymètre lorsque le signal relatif à un mouvement est considéré comme indiquant que l'être vivant est en mouvement.

5. Dispositif d'estimation de la SpO2 selon la revendication 4, dans lequel les moyens pour générer un signal relatif à un mouvement comportent un accéléromètre.

6. Méthode d'estimation de la SpO2 d'un être vivant à partir d'au moins deux signaux photopléthysmographiques, dit signaux PPG, l'un dans le rouge et l'autre dans l'infrarouge, mise en oeuvre par un ordinateur, comportant les étapes :

a) Vérification de la périodicité de chacun des signaux.
b) Calcul d'un indicateur de qualité ($I_R$) d'un coefficient appelé ratio des ratios (RR), le ratio des ratios (RR) étant défini par :

$$RR = \frac{\frac{AC_x}{DC_x}}{\frac{AC_y}{DC_y}}$$

Avec :

- $AC_x$ l'amplitude du signal PPG à la longueur d'onde × nm,
- $DC_x$ la valeur minimale (ou moyenne) du signal PPG à la longueur d'onde × nm.
- $AC_y$ l'amplitude du signal PPG à la longueur d'onde y nm,
- $DC_y$ la valeur minimale (ou moyenne) du signal PPG à la longueur d'onde y nm,

c) Estimation du ratio des ratios (RR) et calcul de la SpO2,
l'étape b) comportant également :

- le calcul d'un indicateur de validité temporel ($V_{T,x}$) de chacun des signaux PPG, l'indicateur de validité ($V_{T,x}$) étant égal à :

$$V_{T,x} = \max_i abs\left(\frac{T_{i,x} - T_s}{T_s}\right)$$

avec $T_i$, x le temps entre deux minimums locaux successifs du signal PPG à la longueur d'onde x, et $T_s$ la période moyenne dudit signal,
- le calcul d'un indicateur de stabilité ($V_{A,x}$) de chacun des signaux PPG, l'indicateur de stabilité étant égal à

$$V_{A,x} = \max_i abs\left(\frac{d_{i,x} - d_{i-1,x}}{d_{i-1,x}}\right)$$

avec $d_{i,x}$ l'amplitude du minimum local pour la période #i correspondant à la longueur d'onde x, et
- la détermination à partir des indicateurs de validité et des indicateurs de stabilité, l'indicateur de qualité($I_R$).

7. Méthode d'estimation de la SpO2 selon la revendication 6, dans laquelle l'indicateur de qualité ($I_R$) est choisi égal à la valeur maximale parmi les valeurs des indicateurs de validité et des indicateurs de stabilité.

8. Méthode d'estimation de la SpO2 selon la revendication 6 ou 7, dans laquelle l'étape c) met en oeuvre un modèle d'estimation du ratio des ratios utilisant des paramètres dont les valeurs sont fixées en fonction de la valeur de l'indicateur de qualité ($I_R$).

9. Méthode d'estimation de la SpO2 selon la revendication 8, dans laquelle l'étape c) met en oeuvre un filtre de Kalman.

10. Méthode d'estimation de la SpO2 selon la revendication 9, dans laquelle le filtre de Kalman suit le modèle :

$$\widehat{RR}_k = \widehat{RR}_{k-1} + n_x$$

$$RR_k = a_k \widehat{RR}_k + n_y$$

Avec

- $RR_k$ le ratio des ratios calculé à parti des valeurs AC et DC mesurées sur le signal PPG,
- $\widehat{RR}_k$ le ratio des ratios estimé et qui sera utilisé pour le calcul de la Sp02,
- $n_x$ un bruit blanc gaussien centré de variance $\sigma_x$ = 0.005,
- $n_y$ un bruit blanc gaussien centré de variance $\sigma_y(k)$,

et dans laquelle les valeurs de $a_k$ et de $\sigma_y(k)$ sont choisis en fonction de la valeur de l'indicateur de qualité.

11. Méthode d'estimation de la SpO2 selon la revendication 10, dans laquelle :

- Si l'indicateur de qualité ($I_R$) <0,1, $\sigma_y(k)$ = 0,05 et $a_k$ = 1,,
- Si 0,1 < l'indicateur de qualité $I_R$ <0,2, $\sigma_y(k)$ = 0.2 et $a_k$ = 1,
- Si 0,2<l'indicateur de qualité ( $I_R$) <0,4, $\sigma_y(k)$ = 0,5 et $a_k$ = 1,
- Si 0,4 < l'indicateur de qualité ($I_R$) <0,8, $\sigma_y(k)$ = 1,0 et $a_k$ = 0,
- Si l'indicateur de qualité ($I_R$) > 0,8, $\sigma_y(k)$ = 2,0 et $a_k$ = 0.

12. Méthode d'estimation de la SpO2 selon l'une des revendications 6 à 11, dans laquelle l'étape a) comporte le calcul de la densité spectrale de puissance de chacun des signaux, la détection d'un seul maximum local et la vérification que ledit maximum local a une amplitude au moins égal à un seuil donné, avantageusement lors de l'étape a), le rythme cardiaque étant déterminé.

13. Méthode d'estimation de la SpO2 selon l'une des revendications 6 à 12, comportant l'enregistrement des signaux PPG préalablement à l'étape a).

**Patentansprüche**

1. Vorrichtung zum Bestimmen des SpO2-Werts eines Lebewesens, umfassend ein Oximeter (2), das dazu bestimmt ist, an dem Lebewesen befestigt zu werden, wobei das Oximeter so konfiguriert ist, dass es mindestens zwei photoplethysmographische Signale, sogenannte PPG-Signale, erzeugt, eines im roten und das andere im infraroten Bereich, und eine Verarbeitungseinheit (4) für die PPG-Signale, wobei die Verarbeitungseinheit (4) ein Mittel zum Überprüfen der Periodizität jedes der PPG-Signale, ein Mittel zum Bestimmen eines Qualitätsindikators ($I_R$) der PPG-Signale, ein Mittel zum Schätzen des Verhältnisses der Verhältnisse (RR), wobei das Verhältnis der Verhältnisse (RR) definiert ist durch:

$$RR = \frac{\frac{AC_x}{DC_x}}{\frac{AC_y}{DC_y}}$$

wobei:

- $AC_x$ die Amplitude des PPG-Signals bei der Wellenlänge $\times$ nm ist,
- $DC_x$ der Mindestwert (oder Durchschnittswert) des PPG-Signals bei der Wellenlänge x nm ist,
- $AC_y$ die Amplitude des PPG-Signals bei der Wellenlänge y nm ist,
- $DC_y$ der Mindestwert (oder Durchschnittswert) des PPG-Signals bei der Wellenlänge y nm ist, und ein Mittel zum Berechnen des SpO2-Werts aus dem geschätzten Wert des Verhältnisses der Verhältnisse (RR) aufweist, wobei das Mittel zum Schätzen den Wert des Qualitätsindikators ($I_R$) beim Schätzen des Verhältnisses der Verhältnisse (RR) berücksichtigt, wobei das Mittel zum Bestimmen eines Qualitätsindikators ($I_R$) der PPG-Signale so konfiguriert ist, dass es einen zeitlichen Gültigkeitsindikator ($V_{T,x}$) jedes der PPG-Signale, wobei der Gültigkeitsindikator ($V_{T,x}$) gleich ist mit:

$$V_{T,x} = \max_i abs\left(\frac{T_{i,x} - T_s}{T_s}\right)$$

wobei $T_{i,x}$ die Zeit zwischen zwei aufeinanderfolgenden lokalen Minima des PPG-Signals bei der Wellenlänge x ist und $T_S$ die durchschnittliche Periode des Signals ist, und einen Stabilitätsindikator ($V_{A,x}$) jedes der PPG-Signale bestimmt, wobei der Stabilitätsindikator gleich ist mit:

$$V_{A,x} = \max_i abs\left(\frac{d_{i,x} - d_{i-1,x}}{d_{i-1,x}}\right)$$

wobei $d_{i,x}$ die Amplitude des lokalen Minimums für die Periode #i entsprechend der Wellenlänge x ist, und den Qualitätsindikator ($I_R$) aus den Gültigkeitsindikatoren und den Stabilitätsindikatoren bestimmt.

2. Vorrichtung zum Schätzen des SpO2-Werts nach Anspruch 1, wobei der Qualitätsindikator ($I_R$) gleich dem Maximalwert unter den Werten der Gültigkeitsindikatoren und der Stabilitätsindikatoren gewählt wird.

3. Vorrichtung zum Schätzen des SpO2-Werts nach Anspruch 1 oder 2, wobei das Mittel zum Überprüfen der Periodizität jedes der PPG-Signale so konfiguriert ist, dass es die spektrale Dichte jedes der PPG-Signale berechnet und die Existenz eines einzelnen lokalen Maximums und den Wert der Amplitude des lokalen Maximums in Bezug auf einen bestimmten Schwellenwert überprüft.

4. Vorrichtung zum Schätzen des SpO2-Werts nach einem der Ansprüche 1 bis 3, umfassend ein Mittel zum Erzeugen eines Signals bezüglich einer Bewegung des Lebewesens und ein Mittel zum Aussetzen der Aktivität des Oximeters, wenn das Signal bezüglich einer Bewegung als Hinweis darauf betrachtet wird, dass das Lebewesen in Bewegung ist.

5. Vorrichtung zum Schätzen des SpO2-Werts nach Anspruch 4, wobei das Mittel zum Erzeugen eines Signals bezüglich einer Bewegung einen Beschleunigungsmesser umfasst.

6. Verfahren zum Schätzen des SpO2-Werts eines Lebewesens aus mindestens zwei photoplethysmographischen Signalen, sogenannten PPG-Signalen, eines im roten und das andere im infraroten Bereich, das von einem Computer durchgeführt wird, umfassend die folgenden Schritte:

a) Überprüfen der Periodizität jedes der Signale,
b) Berechnen eines Qualitätsindikators ($I_R$) eines Koeffizienten, der als Verhältnis der Kennzahlen (RR) bezeichnet wird, wobei das Verhältnis der Kennzahlen (RR) definiert ist durch:

$$RR = \frac{\frac{AC_x}{DC_x}}{\frac{AC_y}{DC_y}}$$

wobei:

- $AC_x$ die Amplitude des PPG-Signals bei der Wellenlänge x nm ist,

- $DC_X$ der Mindestwert (oder Durchschnittswert) des PPG-Signals bei der Wellenlänge x nm ist,
- $AC_Y$ die Amplitude des PPG-Signals bei der Wellenlänge y nm ist,
- $DC_Y$ der Mindestwert (oder Durchschnittswert) des PPG-Signals bei der Wellenlänge y nm ist,

c) Schätzen des Verhältnisses der Kennzahlen (RR) und Berechnen des Sp02-Werts,
wobei Schritt b) auch Folgendes umfasst:

- Das Berechnen eines zeitlichen Gültigkeitsindikators ($V_{T,X}$) jedes der PPG-Signale, wobei der Gültigkeitsindikator ($V_{T,X}$) gleich ist mit:

$$V_{T,x} = \max_i abs \left( \frac{T_{i,x} - T_s}{T_s} \right)$$

wobei $T_{i,x}$ die Zeit zwischen zwei aufeinanderfolgenden lokalen Minima des PPG-Signals bei der Wellenlänge x ist und $T_S$ die durchschnittliche Periode des Signals ist,
- das Berechnen eines Stabilitätsindikators ($V_{A,X}$) jedes der PPG-Signale, wobei der Stabilitätsindikator gleich ist mit

$$V_{A,x} = \max_i abs \left( \frac{d_{i,x} - d_{i-1,x}}{d_{i-1,x}} \right)$$

wobei $d_{i,x}$ die Amplitude des lokalen Minimums für die Periode #i entsprechend der Wellenlänge x ist,
- das Bestimmen des Qualitätsindikators ($I_R$) aus den Gültigkeitsindikatoren und den Stabilitätsindikatoren.

7. Verfahren zum Schätzen des Sp02-Werts nach Anspruch 6, wobei der Qualitätsindikator ($I_R$) gleich dem Maximalwert unter den Werten der Gültigkeitsindikatoren und der Stabilitätsindikatoren gewählt wird.

8. Verfahren zum Schätzen des Sp02-Werts nach Anspruch 6 oder 7, wobei der Schritt c) ein Modell zum Schätzen des Verhältnisses der Kennzahlen unter Verwendung von Parametern, deren Werte entsprechend dem Wert des Qualitätsindikators ($I_R$) festgelegt sind, implementiert.

9. Verfahren zum Schätzen des Sp02-Werts nach Anspruch 8, wobei der Schritt c) ein Kaiman-Filter implementiert.

10. Verfahren zum Schätzen des Sp02-Werts nach Anspruch 9, wobei das Kaiman-Filter dem Modell folgt:

$$\widehat{RR}_k = \widehat{RR}_{k-1} + n_x$$

$$RR_k = a_k \widehat{RR}_k + n_y$$

wobei

- $RR_k$ das Verhältnis der Kennzahlen ist, berechnet aus den am PPG-Signal gemessenen AC- und DC-Werten,
- $\widehat{RR}_k$ das geschätzte Verhältnis der Kennzahlen ist, das zum Berechnen des Sp02-Werts verwendet wird,
- $n_x$ ein Gaußsches Varianzzentrum für weißes Rauschen $\sigma_x = 0,005$ ist,
- $n_y$ ein Gaußsches Varianzzentrum für weißes Rauschen $\sigma_y(k)$ ist,

und wobei die Werte von $a_k$ und $\sigma_y(k)$ entsprechend dem Wert des Qualitätsindikators gewählt werden.

11. Verfahren zum Schätzen des Sp02-Werts nach Anspruch 10, wobei:

- Wenn der Qualitätsindikator ($I_R$) <0,1, $\sigma_y(k)$ = 0,05 und $a_k$ = 1,
- wenn 0,1 < der Qualitätsindikator ($I_R$) <0,2, $\sigma_y(k)$ = 0,2 und $a_k$ = 1,
- wenn 0,2 < der Qualitätsindikator ($I_R$) <0,4, $\sigma_y(k)$ = 0,5 und $a_k$ = 1,
- wenn 0,4 < der Qualitätsindikator ($I_R$) <0,8, $\sigma_y(k)$ = 1,0 und $a_k$ = 0,
- wenn der Qualitätsindikator ($I_R$) > 0,8, $\sigma_y(k)$ = 2,0 und $a_k$ = 0.

12. Verfahren zum Schätzen des Sp02-Werts nach einem der Ansprüche 6 bis 11, wobei der Schritt a) das Berechnen der spektralen Leistungsdichte jedes der Signale, das Erkennen eines einzelnen lokalen Maximums und das Überprüfen, dass das lokale Maximum eine Amplitude aufweist, die mindestens einem bestimmten Schwellenwert entspricht, umfasst, wobei vorteilhafterweise während Schritt a) die Herzfrequenz bestimmt wird.

13. Verfahren zum Schätzen des Sp02-Werts nach einem der Ansprüche 6 bis 12, umfassend das Aufzeichnen der PPG-Signale vor Schritt a).

## Claims

1. A device for evaluating SpO2 of a living being, including an oximeter (2) intended to be fixed on said living being, said oximeter being configured to produce at least two photoplethysmographic signals, called PPG signals, one in the red and the other in the infrared, and a unit (4) for processing said PPG signals, said processing unit (4) including means for checking the periodicity of each of the PPG signals, means for determining a quality indicator ($I_R$) of the PPG signals, means for estimating the ratio of ratios (RR), the ratio of ratios (RR) being defined by:

$$RR = \frac{\dfrac{AC_x}{DC_x}}{\dfrac{AC_y}{DC_y}}$$

With :

- $AC_x$ the amplitude of the PPG signal at the wavelength $\times$ nm,
- $DC_x$ the minimum (or average) value of the PPG signal at the wavelength $\times$ nm.
- $AC_y$ the amplitude of the PPG signal at the wavelength y nm,
- $DC_y$ the minimum (or average) value of the PPG signal at the wavelength y nm, and means for calculating SpO2 from the estimated value of the ratio of ratios (RR), the estimation means taking into account the value of the quality indicator ($I_R$) in the estimation of the ratio of ratios (RR), wherein the means for determining a quality indicator ($I_R$) of the PPG signals are configured to determine a time validity indicator ($V_{T,x}$) of each of the PPG signals, the validity indicator ($V_{T,x}$) being equal to:

$$V_{T,x} = \max_i abs\left(\frac{T_{i,x} - T_s}{T_s}\right)$$

with $T_{i,x}$ the time between two successive local minimums of the PPG signal at the wavelength x, and $T_s$ the average period of said signal, a stability indicator ($V_{A,x}$) of each of the PPG signals, the stability indicator being equal to

$$V_{A,x} = \max_i abs\left(\frac{d_{i,x} - d_{i-1,x}}{d_{i-1,x}}\right)$$

with $d_{i,x}$ the amplitude of the local minimum for the period #i corresponding to the wavelength x, and to determine the quality indicator ($I_R$) from the validity indicators and the stability indicators.

2. The device for estimating SpO2 according to claim 1, wherein the quality indicator ($I_R$) is selected equal to the maximum value from the values of the validity indicators and the stability indicators.

3. The device for estimating SpO2 according to claim 1 or 2, wherein the means for checking the periodicity of each of the PPG signals are configured to calculate the spectral density of each of the PPG signals, to check the existence of a single local maximum and the value of the amplitude of said local maximum relative to a given threshold.

4. The device for estimating SpO2 according to one of claims 1 to 3, including means for generating a signal relating to a movement of said living being and means for suspending the activity of the oximeter when the signal relating to a movement is considered as indicating that the living being is moving.

5. The device for estimating SpO2 according to claim 4, wherein the means for generating a signal relating to a movement include an accelerometer.

6. A method for estimating SpO2 of a living being from at least two photoplethysmographic signals, called PPG signals, one in the red and the other in the infrared, implemented by a computer, including the steps consisting in:

   a) Checking the periodicity of each of the signals.
   b) Calculating a quality indicator ($I_R$) of a coefficient called ratio of ratios (RR), the ratio of ratios (RR) being defined by:

$$RR = \frac{\dfrac{AC_x}{DC_x}}{\dfrac{AC_y}{DC_y}}$$

   With:

   - $AC_x$ the amplitude of the PPG signal at the wavelength x nm,
   - $DC_x$ the minimum (or average) value of the PPG signal at the wavelength $\times$ nm.
   - $AC_y$ the amplitude of the PPG signal at the wavelength y nm,
   - $DC_y$ the minimum (or average) value of the PPG signal at the wavelength y nm,

   c) Estimating the ratio of ratios (RR) and calculating SpO2,
   step b) also including:

- the calculation of a time validity indicator ($V_{T,x}$) of each of the PPG signals, the validity indicator ($V_{T,x}$) being equal to:

$$V_{T,x} = \max_{i} abs\left(\frac{T_{i,x} - T_s}{T_s}\right)$$

with $T_{i,x}$ the time between two successive local minimums of the PPG signal at the wavelength x, and $T_s$ the average period of said signal,
- the calculation of a stability indicator ($V_{A,x}$) of each of the PPG signals, the stability indicator being equal to

$$V_{A,x} = \max_{i} abs\left(\frac{d_{i,x} - d_{i-1,x}}{d_{i-1,x}}\right)$$

with $d_{i,x}$ the amplitude of the local minimum for the period #i corresponding to the wavelength x, and
- the determination of the quality indicator ($I_R$) from the validity indicators and the stability indicators.

7. The method for estimating SpO2 according to claim 6, wherein the quality indicator ($I_R$) is selected equal to the maximum value from the values of the validity indicators and the stability indicators.

8. The method for estimating SpO2 according to claim 6 or 7, wherein step c) implements a model for estimating the ratio of ratios using parameters whose values are fixed depending on the value of the quality indicator ($I_R$).

9. The method for estimating SpO2 according to claim 8, wherein step c) implements a Kalman filter.

10. The method for estimating SpO2 according to claim 9, wherein the Kalman filter follows the model:

$$\widehat{RR_k} = \widehat{RR_{k-1}} + n_x$$

$$RR_k = a_k\widehat{RR_k} + n_y$$

With

- $RR_k$ the ratio of ratios calculated from the AC and DC values measured on the PPG signal,

- $\widehat{RR_k}$ the estimated ratio of ratios and which will be used to calculate SpO2,
- $n_x$ a centred Gaussian white noise of variance

$\sigma_x = 0.005$,
- $n_y$ a centred Gaussian white noise of variance $\sigma_y(k)$,

and wherein the values of $a_k$ and $\sigma_y(k)$ are selected depending on the value of the quality indicator.

11. The method for estimating SpO2 according to claim 10, wherein:

- If the quality indicator ($I_R$) < 0.1, $\sigma_y(k)$ = 0.05 and $a_k$ = 1,
- If 0.1 < the quality indicator ($I_R$) < 0.2, $\sigma_y(k)$ = 0.2 and $a_k$ = 1,
- If 0.2 < the quality indicator ($I_R$) < 0.4, $\sigma_y(k)$ = 0.5 and $a_k$ = 1,
- If 0.4 < the quality indicator ($I_R$) < 0.8, $\sigma_y(k)$ = 1.0 and $a_k$ = 0,
- If the quality indicator ($I_R$) > 0.8, $\sigma_y(k)$ = 2.0 and $a_k$ = 0,

12. The method for estimating SpO2 according to one of claims 6 to 11, wherein step a) includes the calculation of the power spectral density of each of the signals, the detection of a single local maximum and the check that said local maximum has an amplitude which is at least equal to a given threshold, advantageously during step a), the heart rate being determined.

13. The method for estimating SpO2 according to one of claims 6 to 12, including the recording of the PPG signals prior to step a).

FIG.1A

FIG.1B

EP 3 491 999 B1

FIG.1C

FIG.1D

EP 3 491 999 B1

FIG.2

FIG.3A

FIG.3B

FIG.4A

FIG.4B

FIG.5A

FIG.5B

FIG.6A

FIG.6B

FIG.7A

FIG.7B

FIG.8A

FIG.8B

FIG.9A

FIG.9B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2013079601 A
- EP 1611847 A
- US 6725074 B
- US 2017156593 A

**Littérature non-brevet citée dans la description**

- **S. LIU.** Using a Kalman filter to compensate for PPG artefacts when monitoring CT examination. *IEEE,* 2009
- Thermal Measurements and Inverse Techniques. **H. R. B. ORLANDE.** The Eurotherm Seminar 94. Station Biologique de ROSCOFF, 13 Juin 2011